# EUROPEAN PATENT APPLICATION

(11) **EP 4 760 736 A1**
(43) Date of publication of application: **17.06.2026**
(21) Application number: 24218933.0
(22) Date of filing: 11.12.2024
(51) Int. Cl.: G16H 20/30, A61C 5/77, A61C 13/00

(54) **COMPUTER-IMPLEMENTED METHOD AND SYTEM FOR CUSTOMIZING AN INITIAL RESTORATION DESIGN BY A SPECIFIC USER**

(71) Applicant: Dentsply Sirona Inc., York, PA 17401 (US); SIRONA Dental Systems GmbH, 64625 Bensheim (DE)
(72) Inventor: Derzapf, Evgenij, 64625 Bensheim (DE); Löschhorn, Sandro, 64625 Bensheim (DE); Muff, Samuel, 64625 Bensheim (DE); Bielser, Daniel, 64625 Bensheim (DE); Tanaka, Simon, 64625 Bensheim (DE); Koza, André, 64625 Bensheim (DE); Wey, Peter, 64625 Bensheim (DE)
(74) Representative: Aldridge, Henry Alexander

(57) **Abstract**

The present invention relates to a computer-implemented method for customizing an initial restoration design (1) by a specific user, wherein the initial restoration design (1) is received; wherein a digital 3D model (2) of the dental situation of a patient is received. The method further comprises: a first artificial neural network for machine learning adds a generic offset (3) to each region of the initial restoration design (1) taking into account the digital 3D model (2), and outputs the result to a second artificial neural network for machine learning; the second artificial neural network for machine learning adds a user-specific offset (4) to each region of the output of the first artificial neural network taking into account the 3D digital model (2), and outputs the result to a specific user; wherein the specific user, using CAD tools, revises via a display unit the output of the second artificial neural network taking into account the 3D digital model (2) to produce a final restoration design (5); wherein the first artificial neural network is trained using a first training data set, the first training data set including the initial restoration designs (1), the 3D digital models (2), and the final restoration designs (5) produced by at least other users; and wherein the second artificial neural network is trained using a second training data set, the second training data set including initial restoration designs (1), the digital 3D models (2), and final restoration designs (5) produced by the specific user only.

## Description

### TECHNICAL FIELD OF THE INVENTION

The present invention relates to a computer implemented method and system for customizing an initial restoration design by a specific user.

### BACKGROUND ART OF THE INVENTION

Computer aided design (CAD) software for designing dental restoration proposals is generally known in the art. In general, commonly known CAD software has automated workflows which enable a fast and smooth process such that the dentist (hereinafter also referred to as user or specific user) can go from scanning of the dental condition of the patient to manufacturing the dental restoration design, e.g., by using a dental milling machine. Herein the dental milling machine, the intraoral scanner, and the CAD software running on a computer are usually provided as an interconnected system, preferably an IoT (internet of things) System.

The CAD software usually automatically generates a dental restoration design which can be edited by the user by means of a computer GUI in order to design it according to the individual preferences of the dentist. In the state of the art, the CAD software may also learn through use of a neural network from the users, and then design the dental restoration proposal based on what it learned from all users. However, the user often needs a lot of time for post-processing the dental restoration design of the CAD Software according to the individual preferences of the dentist.

### SUMMARY OF THE INVENTION

The inventors are not aware of a system which enables separate machine learning of individual adjustments specific to a user to be made to a dental restoration design, and general adjustments common to at least all other users to be made to a dental restoration design.

An objective of the present invention is to provide a computer implemented method and system for customizing an initial restoration design by a specific user.

This objective has been achieved by the computer-implemented method as defined in claim 1, and the system as defined in claim 7. The subject-matters of the dependent claims define further developments and preferred embodiments.

The computer-implemented method of the present invention is suitable for customizing an initial restoration design by a specific user. According to this method, the initial restoration design is received. And also, a digital 3D model of the dental situation of a patient is received. A first artificial neural network for machine learning adds a generic offset to each region of the initial restoration design taking into account the digital 3D model, and outputs the result to a second artificial neural network for machine learning. The second artificial neural network for machine learning adds a user-specific offset to each region of the output of the first artificial neural network taking into account the 3D digital model, and outputs the result to that specific user. The specific user, using CAD tools, revises via a display the output of the second artificial neural network taking into account the 3D digital model to produce a final restoration design. Herein the first artificial neural network is trained using a first training data set, wherein the first training data set includes the initial restoration designs, the 3D digital models, and the final restoration designs produced by at least the other users. The second artificial neural network is trained using a second training data set, wherein the second training data set includes initial restoration designs, the digital 3D models, and final restoration designs produced by the specific user *only.*

An advantageous effect of the invention is that general learning and the individual learning can be well separated, and thus the post-processing of the design by the specific user can be reduced, while also the influence from other users can be incorporated into the designs. Thereby also the specific learning can better reflect the expectations of the specific-user.

According to the present invention, the initial restoration design can relate to inlay, a crown, a bridge, an abutment crown, a pontic, a veneer, or the like known to those skilled in the art.

According to the present invention the digital 3D model of the of the dental situation can define one or more dental objects such as teeth, abutments for insertion of the final restoration design, and/or color information of the dental situation.

According to the present invention, the CAD tools are digital tools that are operable on a computer and are suitable for adapting e.g., the 3D shape / color of the final restoration design e.g. visually on a display.

The present invention also discloses computer implemented system for customizing an initial restoration design by a specific user. The system can receive an initial restoration design and a digital 3D model of the dental situation of a patient. The system has a processing means for executing the first and second artificial neural networks, and the CAD tools. The system also has a display and input means such as a keyboard, a mouse, and the like for the user input.

According to the present invention the system also comprises manufacturing devices for additively or subtractively manufacturing the final restoration design. The manufacturing device can be a 3D printing unit and a post processing unit for post processing the printed 3D object (design). The manufacturing device can be a milling machine for milling the final restoration design from a dental block.

The present invention also discloses a computer program comprising computer readable codes which can be executed by the system to perform the method. The computer program also allows the user to implement the CAD Tools. The present invention also discloses a computer-readable storage medium which stores the computer program and is accessible to the system.

### BRIEF DESCRIPTION OF THE DRAWINGS

In the following description, the present invention will be explained in more detail with reference to the exemplary embodiments and with reference to the drawings, wherein,
- Fig. 1A -: shows a flowchart of a computer implemented method according to an embodiment of the present invention;
- Fig. 1B -: shows a computer implemented system according to an embodiment of the present invention;
- Fig. 1C -: shows an intraoral scanner used for acquiring a digital impression of a patient according to an embodiment of the present invention;
- Fig. 1D -: shows a milling (dental tool) machine connected with computer implemented system in Fig. 1B;
- Fig. 1E -: shows the milling machine in Fig. 1D, wherein the machining compartment is opened;
- Fig. 1F -: shows an enlarged view of the user interface of the milling machine in Fig. 1D;
- Fig. 1G -: shows various dental tools for the milling machine in Fig. 1D;
- Fig. 2 -: shows an initial restoration design according to an embodiment of the present invention;
- Fig. 3 -: shows a final restoration design according to an embodiment of the present invention;
- Fig. 4a -: shows a square tooth shape according to an embodiment of the present invention;
- Fig. 4b -: shows an ovoid tooth shape according to an embodiment of the present invention;
- Fig. 4c -: shows a tapered tooth shape according to an embodiment of the present invention;
- Fig. 5 -: shows an initial restoration design and a proximal contact region with a generic offset according to an embodiment of the present invention;
- Fig. 6 -: shows an initial restoration design and a proximal contact region with a user specific offset according to an embodiment of the present invention;
- Fig. 7 -: shows an occlusal contact region according to an embodiment of the present invention;
- Fig. 8 -: shows a proximal contact region according to an embodiment of the present invention;
- Fig. 9 -: shows a slope preparation margin according to an embodiment of the present invention.

The reference numbers shown in the drawings designate the elements listed below, which are referred to in the following description of the exemplary embodiments.
- 1.: Initial restoration design
- 1a.: Crown
- 2.: Digital 3D model
- 2a.: Adjacent tooth,
- 3.: Generic offset
- 4.: User-specific offset
- 5.: Final restoration design
- 6.: Squared tooth
- 7.: Ovoid tooth
- 8.: Tapered tooth
- 9.: Occlusal contact region
- 10.: Proximal contact region,
- 11.: Preparation margin region
- 12.: Computer implemented System.
- 13.: Display
- 14.: Processing means (Computer)
- 15.: MRI device
- 16.: 3D printing unit
- 17.: Post processing unit
- 18.: Intraoral optical scanner
- 19.: Milling machine
- 20.: Dental Block
- 21.: Dental Tool
- 22.: Driving unit (Carriage)
- 23.: User interface (e.g., Touch screen)

### DETAILED DESCRIPTION OF THE PREFERRED EMBODIMENTS

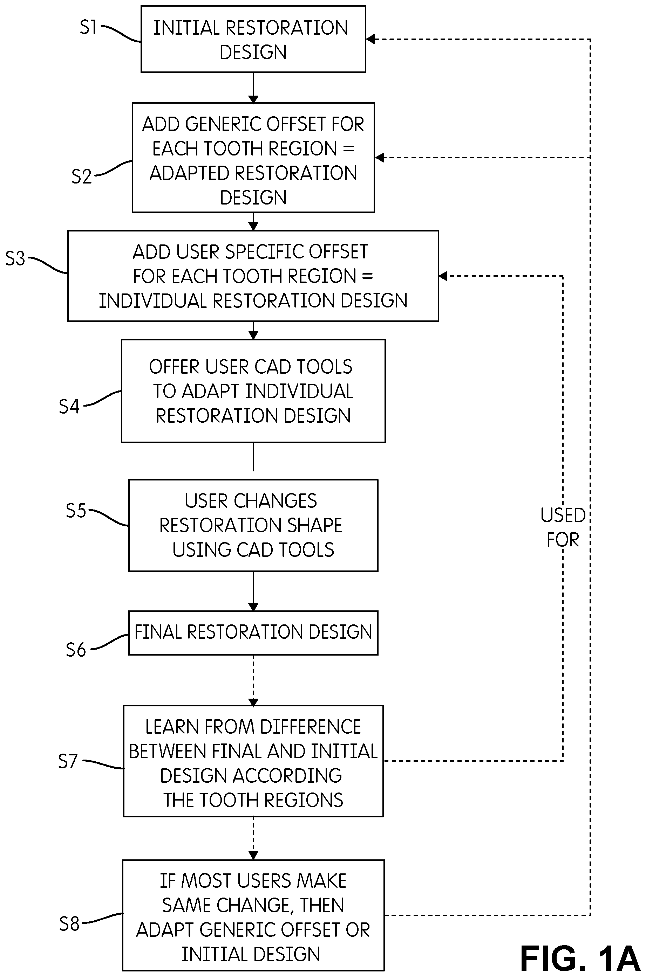

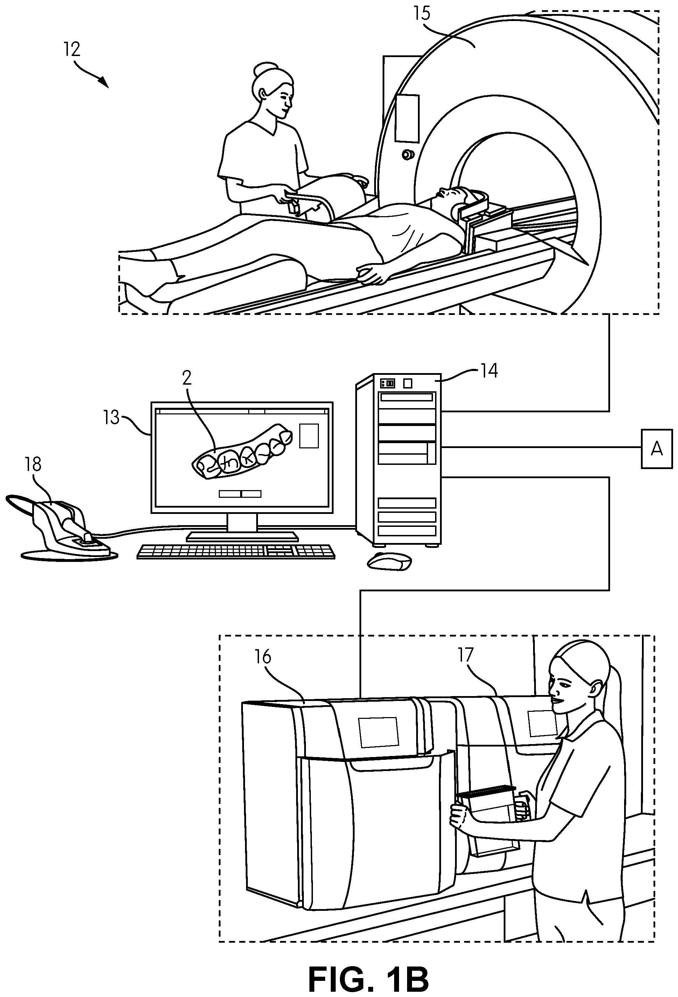

Fig. 1A shows a flowchart of a method according to an embodiment. The method will be explained in more detail below. The method is a computer-implemented method (i.e., herein also referred to as a software) and can be carried out on a computer-implemented system (12) as shown in an embodiment in Fig. 1B.

The present invention also includes a corresponding computer program (i.e., the software) having computer-readable code for implementing the method. The computer program is provided on a computer readable storage medium accessible to the system (12).

The computerized system (12) shown in Fig. 1B is a multifunctional system for acquiring 2D images, 3D surface images, Volume images of the dental condition of the patient. The system (12) has a computer (14), a display (13), and input means such as keyboard and mouse. The computer (14) can be connected to various imaging devices such as a handheld optical intraoral scanner (18), a dental dedicated magneto resonance imaging device (15), or an X-ray imaging device with DVT/PAN/CEPH modalities (not shown).

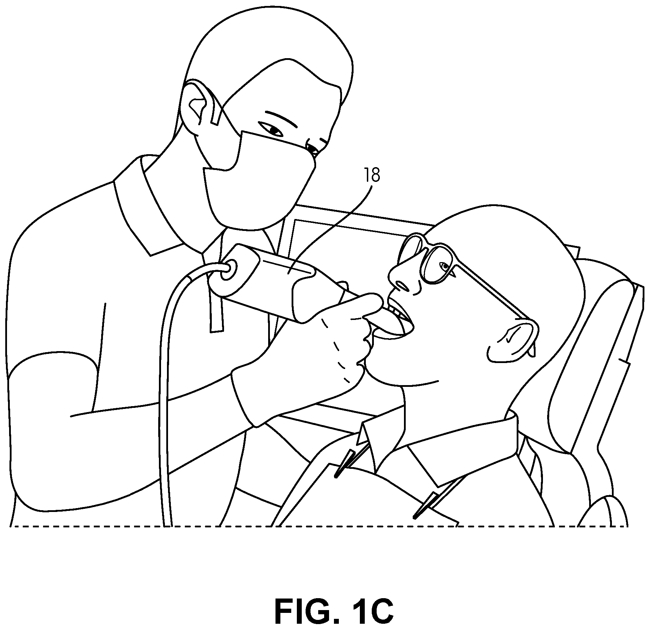

As shown in Fig. 1C, the intraoral scanner (18) can be used for acquiring a digital impression of a patient. Also, a wireless intraoral scanner can be used and connected to the system via wireless communication. The digital impression is a 3D surface image of the dental condition. The digital impression can serve to generate a digital 3D model (2). Other imaging modalities such MR imaging, X-ray imaging can also be used for generating digital 3D models (2).

The system (1) is preferably configurable as an IoT (internet of things) system for cloud computing, data exchange, remote control, and the like, wherein the computer (14) can be bidirectionally connected via a local area network and/or the internet (not shown) to other dental devices (not shown) such as the optical intraoral scanner (18), the MRI device (15), an X-ray imaging device (not shown), a dental milling machine (19), a 3D printing unit (16), a post processing units (17), and data storages (not shown). The system (12) can be used by multiple users such as dentists.

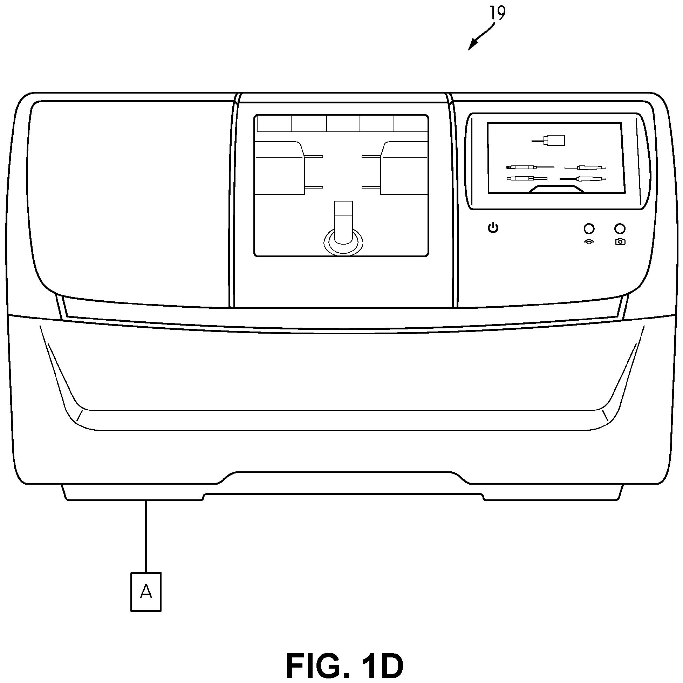

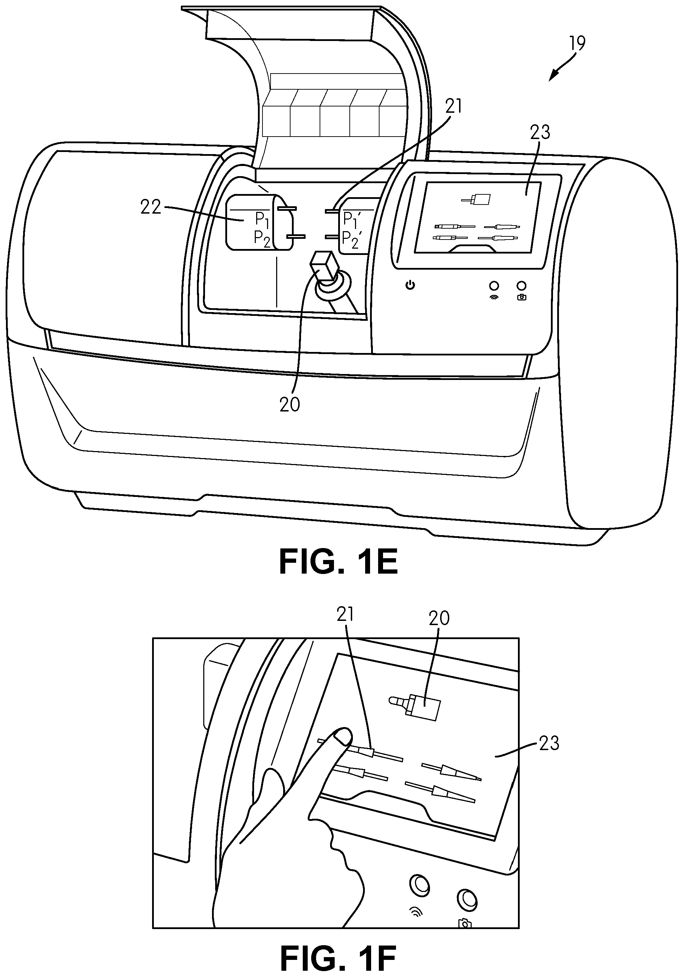

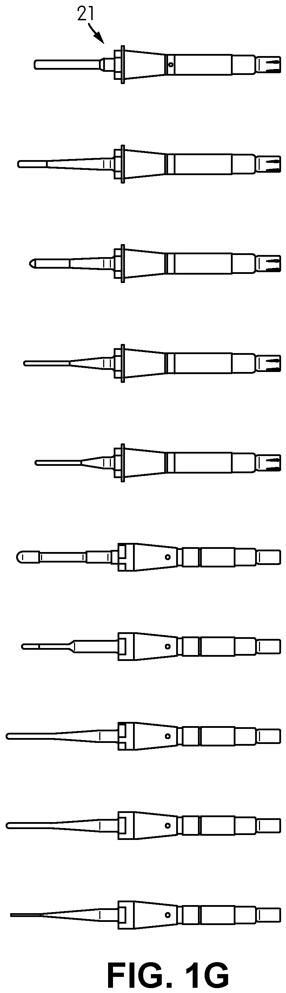

Fig. 1D and fig. 1E show the milling machine (19) for manufacturing a dental restoration design. The milling machine (19) comprises: a dental blank holder which holds a dental block (20) relatively movable with respect to the dental tools (21); two driving units (22) each movably holding one or more dental tools (21) for machining the dental blank (20); and a control unit adapted to control the dental blank holder and the driving units (22) based at least on a trajectory of the dental tools (21) relative to the dental blank (20) and a spatial amount of material removal from the dental blank (2) along the trajectory. As shown in Fig. 1F, the milling machine (19) also has a user interface (touch screen). As shown in Fig. 1G various different dental tools (21) can be used to process the dental blank (20). The dental tools (21) may have micro-RFID tags at their rear side in a housing which can be recognized by the RFID reader/writer of the milling machine (19).

Fig. 1B shows a 3D printing unit (3D printer) according to one embodiment of the invention. The 3D printer according to the invention comprises a vat with an at least partially transparent bottom for receiving the liquid photoreactive resin for producing a solid component; a building platform for pulling out the component layer by layer out from the vat; a projector for projecting the layer geometry onto the transparent base; a transport apparatus for moving the building platform downward or upward in the vat. The 3D printer has a computer-implemented control unit (not shown) for controlling the overall operation. Fig. 1B also shows a post processing unit for post processing such as washing/drying/post exposure of the 3D objects printed by the (3D printer).

The software according to the invention allows high-quality automatic preparation of 3D printing or milling jobs for dental components such as splints, denture bases, models, restoration designs such as bridges and crowns and the like.

In the subsequent the flowchart in Fig. 1A will be explained in more detail. The computer-implemented method is suitable for customizing an initial restoration design (1) by a specific user by using the system (12).

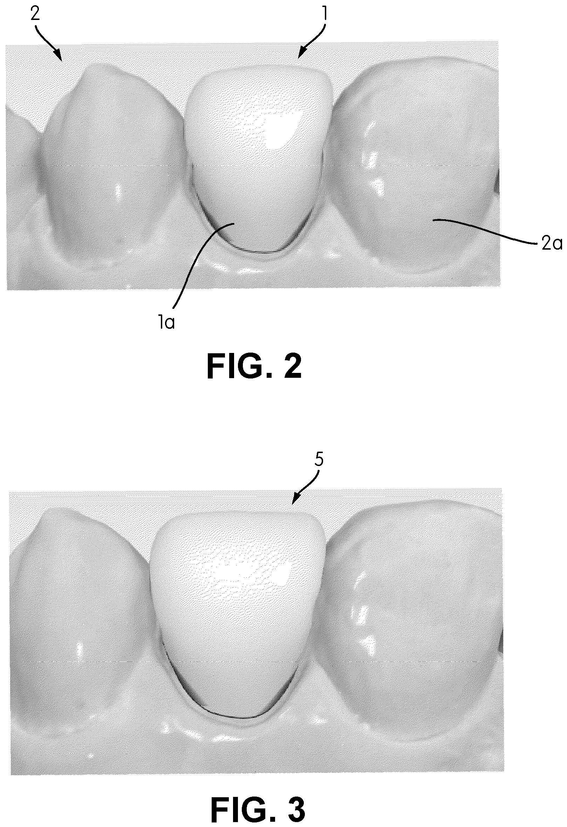

In step S1 an initial restoration design (1) and a digital 3D model (2) of the dental situation of a patient is received. The system (12) can receive the initial restoration design (1) form an external source or generate this based on the dental situation of the patient. Similarly, the system (12) can receive the digital 3D model (2) from an external source or generate this based on imaging data such as a dental impression or the like. An exemplary initial restoration design (1) is shown in Fig. 2. According to various embodiments, the initial restoration design (1) can be an inlay, a crown (1a), a bridge, an abutment crown, a pontic, or a veneer. An exemplary digital 3D model (2) of the of the dental situation is shown also in Fig 2. According to various embodiments, the digital 3D model (2) can comprise at least one prepared tooth, at least one adjacent tooth (2a), at least one residual tooth, at least one abutment for insertion of the final restoration design (5), and/or color information of the dental situation.

The system (12) has a first artificial neural network and a second artificial neural network which will be later described later in more detail.

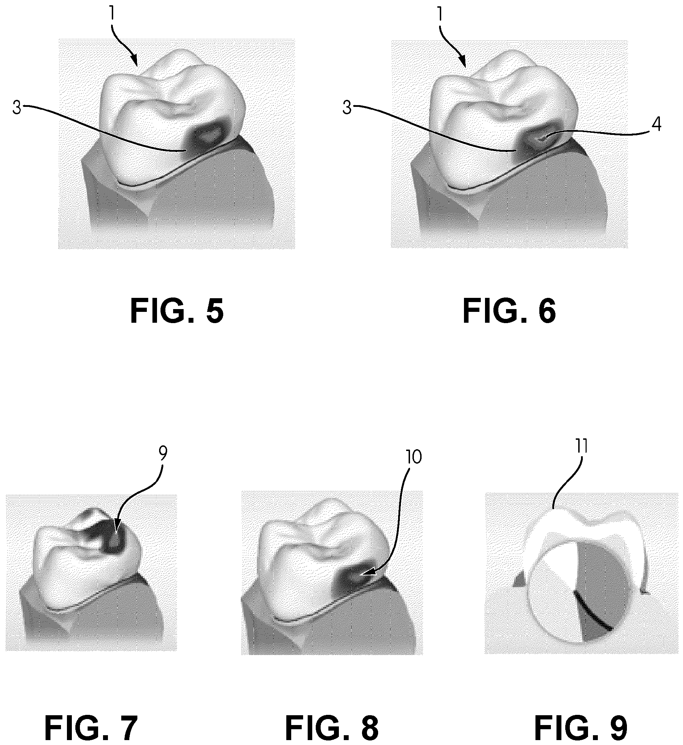

In step S2, the first artificial neural network for machine learning adds a generic offset (3) to each region of the initial restoration design (1) taking into account the digital 3D model (2), and outputs the result to a second artificial neural network for machine learning. Fig. 5 shows an exemplary initial restoration design (1) with a proximal contact region (10) to which a generic offset (3) has been accordingly added.

In step S3, the second artificial neural network for machine learning adds a user-specific offset (4) to each region of the output of the first artificial neural network taking into account the 3D digital model (2), and outputs the result to the specific user. Fig. 6 shows an exemplary initial restoration design (1) with a proximal contact region (10) to which a user specific offset (4) has been accordingly added.

In step S4, the specific user, using CAD tools, revises via the display (13) the output of the second artificial neural network taking into account the 3D digital model (2) to produce a final restoration design (5). Fig. 3 shows an exemplary final restoration design (5) revised by the user accordingly.

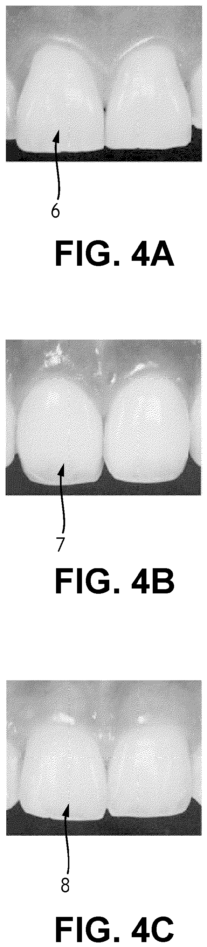

The CAD Tools are for adapting the 3D shape of the final restoration design (5). In an embodiment, the CAD Tool is a tooth shape tool for producing a squared tooth (6), an ovoid tooth (7), or a tapered tooth (8) shape. Fig. 4a shows an exemplary square tooth shape. Fig. 4b shows an exemplary ovoid tooth shape. Fig. 4c shows an exemplary tapered tooth shape. The CAD Tool can be a Biogeneric shape tool for changing the morphology of the tooth. The CAD Tool can be a form tool for adding material to a tooth or removing material from a tooth or for smoothing a tooth. The CAD Tool can be a positioning tool for positioning, rotating or scaling a tooth. The CAD Tool can be an incisal shape tool for adapting anterior tooth restorations by adding microstructures. The CAD Tool can be a contact tool for creating contacts to either the mesial or distal neighbor tooth.

In step S4, the specific user may be presented with several individual restoration designs and can then decide on the best proposal from his/her point of view. The selection does not necessarily have to be made for the entire restoration, but can also be made per area (i.e,, Tooth region) or criterion, e.g. only occlusal. An example of a tool would be a slider where one could switch between different proposals.

In the above examples, said region of the initial restoration design (1) are not limited to the proximal contact region (10) as shown in Fig. 10. The initial restoration design (1) may also include an occlusal contact region (9) as shown in Fig. 7. The initial restoration design (1) may also include a labial contact region, a buccal contact region, a lingual contact region, and/or a tooth neck region. The initial restoration design (1) may also have a region close to a preparation margin (11) as shown in Fig. 9.

According to the method, the initial restoration design (1) is divided into several areas/criteria (e.g. occlusal area, proximal area, slope on the preparation margin criteria, contact situation criteria) which do or do not overlap. The overall shape of the initial restoration design (1) results from the shapes of the different areas (i., tooth region).

In step S5, the final restoration design (5) is output by the system (12) and can be manufactured by the dental milling machine (19) or the 3D printing unit (16). The post processing units (17) can be used for prost processing any printed 3D objects for washing, drying, UV curing.

According to the present invention the system performs a continuous learning of changes between final restoration design and initial restoration design. The flowchart in Fig. 1A shows the learning presses in step S7 and step S8.

The system has a first artificial neural network and a second artificial neural network. The first and second artificial neural networks for machine learning can be Convolutional Neural Networks (CNN), Recurrent Neural Networks (RNN) or MultiLayer Perceptrons (MLP).

In step S7, the first artificial neural network is trained by using a first training data set. The first training data set includes the initial restoration designs (1), the 3D digital models (2), and the final restoration designs (5) produced by at least other users.

In step S8, the second artificial neural network is trained by using a second training data set. The second training data set includes the initial restoration designs (1), the digital 3D models (2), and final restoration designs (5) produced by the specific user *only.*

According to the learning method the areas/criteria between the initial restoration design and the final restoration design are compared to determine differences.

According to the learning method, user data is collected centrally and distinguished if it is an adaptation specific to the user or if many users make the same adaptation so that a generic adaptation can be made (i.e., adapt generic offset) or the initial restoration design can be adapted.

According to the learning method, the generic and user specific adaptations are done by weighting (i.e. the more often a certain adaptation takes place the more it will have influence). These adaptions can be material removal, material addition, change of shape, change of texture (smoothness/roughness), change contact situation of neighboring teeth (i.e., contact area/strength), and/or change of material color.

Users may have different preferences for the individual areas/criteria. During the learning, distinction should be made between individual preference for anterior and posterior teeth. What has been learned is preferably mapped to the specific restoration type (e.g. crown).

## Claims

1. A computer-implemented method for customizing an initial restoration design (1) by a specific user,
wherein the initial restoration design (1) is received;
wherein a digital 3D model (2) of the dental situation of a patient is received,
**characterized in that**
a first artificial neural network for machine learning adds a generic offset (3) to each region of the initial restoration design (1) taking into account the digital 3D model (2), and outputs the result to a second artificial neural network for machine learning;
the second artificial neural network for machine learning adds a user-specific offset (4) to each region of the output of the first artificial neural network taking into account the 3D digital model (2), and outputs the result to a specific user;
the specific user, using CAD tools, revises via a display the output of the second artificial neural network taking into account the 3D digital model (2) to produce a final restoration design (5);
wherein the first artificial neural network is trained using a first training data set, the first training data set including the initial restoration designs (1), the 3D digital models (2), and the final restoration designs (5) produced by at least other users; and
wherein the second artificial neural network is trained using a second training data set, the second training data set including initial restoration designs (1), the digital 3D models (2), and final restoration designs (5) produced by the specific user only.

2. The computer-implemented method according to claim 1, **characterized in that** the initial restoration design (1) is an inlay, a crown (1a), a bridge, an abutment crown, a pontic, or a veneer.

3. The computer-implemented method according to claim 1 or 2, **characterized in that** the digital 3D model (2) of the of the dental situation comprises at least one of the following:
- at least one prepared tooth,
- at least one adjacent tooth (2a),
- at least one residual tooth,
- at least one abutment for insertion of the final restoration design (5), and
- color information of the dental situation.

4. The computer-implemented method according to any one of the preceding claims, **characterized in that** the CAD Tools are for adapting the 3D shape of the final restoration design (5), wherein the CAD Tools comprise one or more of the following:
- Tooth shape tool for producing a squared tooth (6), an ovoid tooth (7), or a tapered tooth (8) shape,
- Biogeneric shape tool for changing the morphology of the tooth,
- Form tool for adding material to a tooth or removing material from a tooth or for smoothing a tooth,
- Positioning tool for positioning, rotating or scaling a tooth,
- Incisial shape tool for adapting anterior tooth restorations by adding microstructures,
- Contact tool for creating contacts to either the mesial or distal neighbor tooth.

5. The computer-implemented method according to any one of the preceding claims, **characterized in that** the said region of the initial restoration design (1) comprise one or more the following:
- occlusal contact region (9)
- proximal contact region (10),
- labial contact region,
- buccal contact region,
- lingual contact region,
- tooth neck region,
- region close to a preparation margin (11).

6. The computer-implemented method according to any one of the preceding claims, **characterized by** further comprising a step of manufacturing the final restoration design (5).

7. A computer implemented system (12) for customizing an initial restoration design (1) by a specific user, comprising:
a receiving means for receiving an initial restoration design (1) and a digital 3D model (2) of the dental situation of a patient;
a display unit (13);
processing means (14) **characterized by** comprising:
a first artificial neural network for machine learning that is adapted to add a generic offset (3) to each region of the initial restoration design (1) taking into account the digital 3D model (2), and output the result to a second artificial neural network for machine learning;
the second artificial neural network for machine learning that is adapted to add a user-specific offset (4) to each region of the output of the first artificial neural network taking into account the 3D digital model (2), and output the result to the specific user;
CAD tools for enabling the specific user to revise, via the display unit (13), the output of the second artificial neural network taking into account the 3D digital model (2) to produce a final restoration design (5);
wherein processing means (14) is adapted to train
the first artificial neural network using a first training data set, wherein the first training data set includes the initial restoration designs (1), the 3D digital models (2), and the final restoration designs (5) produced by at least other users; and
wherein processing means (14) is adapted to train
the second artificial neural network using a second training data set, wherein the second training data set includes initial restoration designs (1), the digital 3D models (2), and final restoration designs (1) produced by the specific user only.

8. The computer implemented system (12) according to claim 7, **characterized in that** the initial restoration design (1) is an inlay, a crown (1a), a bridge, an abutment crown, a pontic, or a veneer.

9. The computer implemented system (12) according to claim 7 or 8, **characterized in that** the digital 3D model (2) of the of the dental situation comprises at least one of the following:
- at least one prepared tooth,
- at least one adjacent tooth (2a),
- at least one residual tooth,
- at least one abutment for insertion of the final restoration design (5), and
- color information of the dental situation.

10. The computer implemented system (12) according to any one of claims 7 to 9, **characterized in that** the CAD Tools are for adapting the 3D shape of the final restoration design (5), wherein the CAD Tools comprise one or more of the following:
- Tooth shape tool for producing a tapered tooth (8), an ovoid tooth (7), or a squared tooth (6) shape,
- Biogeneric shape tool for changing the morphology of the tooth,
- Form tool for adding material to a tooth or removing material from a tooth or for smoothing a tooth,
- Positioning tool for positioning, rotating or scaling a tooth,
- Incisial shape tool for adapting anterior tooth restorations by adding microstructures,
- Contact tool for creating contacts to either the mesial or distal neighbor tooth.

11. The computer implemented system (12) according to any one of the claims 7 to 10, **characterized in that** the said region of the initial restoration design comprise one or more the following:
- occlusal contact region (9),
- proximal contact region (10),
- labial contact region,
- buccal contact region,
- lingual contact region,
- tooth neck region,
- region close to a preparation margin (11).

12. The computer implemented system (12) according to any one of the claims 7 to 11, **characterized by** further comprising manufacturing device (16,17; 19) for manufacturing the final restoration design (5).

13. A computer program comprising computer readable codes which, when the computer program is executed by a computer implemented system (12), causes said system (12) to execute the method according to any one of claims 7 to 12.

14. A computer-readable storage medium which stores the computer program according to claim 13.

## Amended claims

### Amended claims in accordance with Rule 137(2) EPC.

1. A computer-implemented method for customizing an initial restoration design (1) by a specific user,
wherein the initial restoration design (1) is received;
wherein a digital 3D model (2) of the dental situation of a patient is received,
**characterized in that**
a first artificial neural network for machine learning adds a generic offset (3) to a region of the initial restoration design (1) taking into account the digital 3D model (2), and outputs the result to a second artificial neural network for machine learning;
the second artificial neural network for machine learning adds a user-specific offset (4) to a region of the output of the first artificial neural network taking into account the 3D digital model (2), and outputs the result to a specific user;
the specific user, using CAD tools, revises via a display the output of the second artificial neural network taking into account the 3D digital model (2) to produce a final restoration design (5);
wherein the first artificial neural network is trained using a first training data set, the first training data set including the initial restoration designs (1), the 3D digital models (2), and the final restoration designs (5) produced by at least other users; and
wherein the second artificial neural network is trained using a second training data set, the second training data set including initial restoration designs (1), the digital 3D models (2), and final restoration designs (5) produced by the specific user only.

2. The computer-implemented method according to claim 1, **characterized in that** the initial restoration design (1) is an inlay, a crown (1a), a bridge, an abutment crown, a pontic, or a veneer.

3. The computer-implemented method according to claim 1 or 2, **characterized in that** the digital 3D model (2) of the of the dental situation comprises at least one of the following:
- at least one prepared tooth,
- at least one adjacent tooth (2a),
- at least one residual tooth,
- at least one abutment for insertion of the final restoration design (5), and
- color information of the dental situation.

4. The computer-implemented method according to any one of the preceding claims, **characterized in that** the CAD Tools are for adapting the 3D shape of the final restoration design (5), wherein the CAD Tools comprise one or more of the following:
- Tooth shape tool for producing a squared tooth (6), an ovoid tooth (7), or a tapered tooth (8) shape,
- Biogeneric shape tool for changing the morphology of the tooth,
- Form tool for adding material to a tooth or removing material from a tooth or for smoothing a tooth,
- Positioning tool for positioning, rotating or scaling a tooth,
- Incisial shape tool for adapting anterior tooth restorations by adding microstructures,
- Contact tool for creating contacts to either the mesial or distal neighbor tooth.

5. The computer-implemented method according to any one of the preceding claims, **characterized in that** the said region of the initial restoration design (1) comprises one or more the following:
- occlusal contact region (9)
- proximal contact region (10),
- labial contact region,
- buccal contact region,
- lingual contact region,
- tooth neck region,
- region close to a preparation margin (11).

6. The computer-implemented method according to any one of the preceding claims, **characterized by** further comprising a step of manufacturing the final restoration design (5).

7. A computer implemented system (12) for customizing an initial restoration design (1) by a specific user, comprising:
a receiving means for receiving an initial restoration design (1) and a digital 3D model (2) of the dental situation of a patient;
a display unit (13);
processing means (14) **characterized by** comprising:
a first artificial neural network for machine learning that is adapted to add a generic offset (3) to a region of the initial restoration design (1) taking into account the digital 3D model (2), and output the result to a second artificial neural network for machine learning;
the second artificial neural network for machine learning that is adapted to add a user-specific offset (4) to a region of the output of the first artificial neural network taking into account the 3D digital model (2), and output the result to the specific user;
CAD tools for enabling the specific user to revise, via the display unit (13), the output of the second artificial neural network taking into account the 3D digital model (2) to produce a final restoration design (5);
wherein processing means (14) is adapted to train
the first artificial neural network using a first training data set, wherein the first training data set includes the initial restoration designs (1), the 3D digital models (2), and the final restoration designs (5) produced by at least other users; and
wherein processing means (14) is adapted to train
the second artificial neural network using a second training data set, wherein the second training data set includes initial restoration designs (1), the digital 3D models (2), and final restoration designs (1) produced by the specific user only.

8. The computer implemented system (12) according to claim 7, **characterized in that** the initial restoration design (1) is an inlay, a crown (1a), a bridge, an abutment crown, a pontic, or a veneer.

9. The computer implemented system (12) according to claim 7 or 8, **characterized in that** the digital 3D model (2) of the of the dental situation comprises at least one of the following:
- at least one prepared tooth,
- at least one adjacent tooth (2a),
- at least one residual tooth,
- at least one abutment for insertion of the final restoration design (5), and
- color information of the dental situation.

10. The computer implemented system (12) according to any one of claims 7 to 9, **characterized in that** the CAD Tools are for adapting the 3D shape of the final restoration design (5), wherein the CAD Tools comprise one or more of the following:
- Tooth shape tool for producing a tapered tooth (8), an ovoid tooth (7), or a squared tooth (6) shape,
- Biogeneric shape tool for changing the morphology of the tooth,
- Form tool for adding material to a tooth or removing material from a tooth or for smoothing a tooth,
- Positioning tool for positioning, rotating or scaling a tooth,
- Incisial shape tool for adapting anterior tooth restorations by adding microstructures,
- Contact tool for creating contacts to either the mesial or distal neighbor tooth.

11. The computer implemented system (12) according to any one of the claims 7 to 10, **characterized in that** the said region of the initial restoration design comprises one or more the following:
- occlusal contact region (9),
- proximal contact region (10),
- labial contact region,
- buccal contact region,
- lingual contact region,
- tooth neck region,
- region close to a preparation margin (11).

12. The computer implemented system (12) according to any one of the claims 7 to 11, **characterized by** further comprising manufacturing device (16,17; 19) for manufacturing the final restoration design (5).

13. A computer program comprising computer readable codes which, when the computer program is executed by a computer implemented system (12), causes said system (12) to execute the method according to any one of claims 7 to 12.

14. A computer-readable storage medium which stores the computer program according to claim 13.
